# FASCICULE DE BREVET EUROPEEN

(11) **EP 3 927 350 B1**
(45) Date de publication et mention de la délivrance du brevet: **22.05.2024**
(21) Numéro de dépôt: 20713705.0
(22) Date de dépôt: 21.02.2020
(51) Int. Cl.: A61K 33/06, A61P 21/00

(54) **SUPPLÉMENT À BASE DE MAGNÉSIUM POUR VOLAILLES**
MAGNESIUMERGÄNZUNG FÜR GEFLÜGEL
MAGNESIUM SUPPLEMENT FOR POULTRY

(30) Priorité: 21.02.2019 FR 1901782
(43) Date de publication de la demande: 29.12.2021
(73) Titulaire: Timab Magnesium, 35800 Dinard (FR)
(72) Inventeur: SCHNEEGANS, Marie-Sophie, 35800 Dinard (FR); DUCLOS, Julie, 35780 La Richardais (FR)
(74) Mandataire: Cabinet Beau de Loménie
(86) Numéro de dépôt international: PCT/FR2020/050333
(87) Numéro de publication internationale: WO 2020/169937

(56) Documents cités:
- LLADOS ET AL: "Neurogenic origin of an experimental myopathy induced by the ionophore A23187", JOURNAL OF NEUROLOGICAL SCIENCES, ELSEVIER SCIENTIFIC PUBLISHING CO, AMSTERDAM, NL, vol. 69, no. 3, 1 juillet 1985 (1985-07-01), pages 171-182, XP024300498, ISSN: 0022-510X, DOI: 10.1016/0022-510X(85)90131-5 [extrait le 1985-07-01]
- SHASTAK Y ET AL: "A review of the role of magnesium in poultry nutrition", WORLDS POULTRY SCIENCE JOURNAL, vol. 71, no. 1, mars 2015 (2015-03), pages 125-137, XP009517183, ISSN: 0043-9339
- J. NELSON: FEEDSTUFFS, vol. 87, no. 23, 22 juin 2015 (2015-06-22) , XP009517191, cité dans la demande
- GUO YUMING ET AL: "Effects of source and level of magnesium and Vitamin E on prevention of hepatic peroxidation and oxidative deterioration of broiler meat.", ANIMAL FEED SCIENCE AND TECHNOLOGY, vol. 107, no. 1-4, 30 juin 2003 (2003-06-30), pages 143-150, XP002795712, ISSN: 0377-8401
- DATABASE BIOSIS [Online] BIOSCIENCES INFORMATION SERVICE, PHILADELPHIA, PA, US; March 2015 (2015-03), SHASTAK Y ET AL: "A review of the role of magnesium in poultry nutrition", Database accession no. PREV201500519116 & WORLDS POULTRY SCIENCE JOURNAL, vol. 71, no. 1, March 2015 (2015-03), pages 125-137, ISSN: 0043-9339(print), DOI: 10.1017/S0043933915000112
- DATABASE BIOSIS [Online] BIOSCIENCES INFORMATION SERVICE, PHILADELPHIA, PA, US; 30 June 2003 (2003-06-30), GUO YUMING ET AL: "Effects of source and level of magnesium and Vitamin E on prevention of hepatic peroxidation and oxidative deterioration of broiler meat.", Database accession no. PREV200300365119 & GUO YUMING ET AL: "Effects of source and level of magnesium and Vitamin E on prevention of hepatic peroxidation and oxidative deterioration of broiler meat.", ANIMAL FEED SCIENCE AND TECHNOLOGY, vol. 107, no. 1-4, 30 June 2003 (2003-06-30), pages 143-150, ISSN: 0377-8401

## Description

### DOMAINE TECHNIQUE

L'invention se rapporte à un additif alimentaire pour son utilisation pour diminuer l'apparition des myopathies dans les élevages de volailles. Ce produit contient au moins une source de magnésium.

### ART ANTERIEUR

Au cours des dernières décennies, la sélection génétique de certaines lignées et la mise en place de programmes d'alimentation améliorés ont augmenté la vitesse de croissance des volailles et, par conséquent, raccourci le cycle de production. Toutefois, cette amélioration a été obtenue au détriment de la qualité de la viande de volaille.

L'industrie avicole est actuellement confrontée à un défi majeur lié aux myopathies émergentes se manifestant par la présence de bandes ou de stries blanches parallèles aux fibres musculaires (« white stripping »), mais également par la dureté de la viande (appelée « wooden breast » dans le cas des filets). Ces deux myopathies touchent plus fréquemment les filets de poulets (Pectolaris major) mais peuvent également être observées dans les muscles des cuisses. Leur incidence augmente avec le poids des volailles c'est pourquoi elles sont plus fréquemment observées chez les volailles lourdes à fort rendement musculaire (typiquement les poulets dont le poids d'abattage est supérieur à 2.4 kg en moyenne). Elles ne présentent pas de risques pour la consommation humaine cependant elles impactent fortement la composition de la viande (baisse de la teneur en protéines et augmentation de la teneur en lipides) ainsi que les propriétés technologiques de la viande, comme la diminution de la rétention en eau.

Ces myopathies conduisent à un taux de déclassement par lot élevé, voire, pour les filets présentant des degrés de sévérité trop importants, à l'absence de commercialisation.

Différentes solutions passant par la gestion de l'alimentation ont donc été proposées et commercialisées pour réduire leur incidence.

C'est le cas de divers extraits de plantes avec des actions antioxydantes, anti-inflammatoires et/ou de stimulation de la circulation sanguine pour une meilleure oxygénation des cellules musculaires des animaux, et d'un mélange comprenant un antioxydant et un oligoélément (zinc, cuivre ou manganèse) chélaté avec de la méthionine. L'effet positif de ce dernier sur la diminution du nombre de poulets souffrant d'une dureté de filet d'indice 2 ou 3 a été démontré en prenant comme référence une supplémentation équivalente en oligoéléments non chélatés. Ces additifs agissent sur l'inflammation, une fois les cellules musculaires entrées en stress oxydatif.

Subsiste le besoin de pouvoir améliorer la qualité de la viande de volaille en agissant en amont de la réaction anti-inflammatoire de manière à améliorer le bien-être de l'animal. Subsiste également le besoin de proposer des additifs pour lutter contre les myopathies sans réduire les performances de croissance des volailles.

Le supplément alimentaire pour volailles de l'invention répond à ce besoin et comprend au moins une source de magnésium.

L'impact de la supplémentation en magnésium a déjà fait l'objet d'une étude sur les volailles (J. Nelson, Feedstuffs, Vol. 87, No. 23, June 22, 2015). Il a été démontré en particulier que le magnésium augmente le poids vif des poulets tout en diminuant l'indice de conversion de l'aliment. Un effet bénéfique sur la solidité des os a également été mis en évidence. L'addition de magnésium chez les poulettes et les poules pondeuses augmente le poids vif, la résistance osseuse ainsi que le poids des oeufs en période de fin de ponte.

Des additifs alimentaires comprenant du magnésium ont par ailleurs été décrits. Par exemple, un additif contenant du lysinate de magnésium en combinaison avec d'autres actifs tels que des extraits végétaux, des chélates de zinc, fer, cuivre, manganèse, de la lysine, des microorganismes et de la vitamine E augmente la croissance, améliore la qualité de la viande et des oeufs, et renforce le système immunitaire (CN 107518202A). D'autres additifs comprenant du sulfate de magnésium en combinaison avec d'autres minéraux, des protéines, des acides, des antibactériens, des extraits végétaux, et/ou du glucose permettent de diminuer l'indice de consommation et la digestibilité de l'aliment (CN 107136318A, CN 106376727A et CN 106798148A).

Llados et al. ont proposé dans l'article paru dans le Journal of Neurological Sciences, 69, 3, 1985, 171-182, un modèle de myopathie in vitro sur un muscle de grenouille, qui consiste à ajouter l'ionophore *A23187* dans un milieu d'incubation contenant des ions calcium.

Or, les inventeurs ont découvert de façon très surprenante que le magnésium permet de réduire l'apparition des myopathies dans les élevages de volaille, sans réduire les performances de croissance des animaux.

### DESCRIPTION DE L'INVENTION

L'invention a donc pour objet un supplément alimentaire comprenant au moins une source de magnésium choisie parmi les sels, les hydrates ou les chélates du magnésium pour son utilisation dans la prévention des myopathies chez les volailles d'élevage.

Selon un mode de réalisation, le supplément est constitué d'au moins une source de magnésium. Ledit supplément peut être constitué d'oxyde de magnésium.

La supplémentation en magnésium des volailles permet avantageusement, dans un rôle préventif, de diminuer l'incidence de survenue des myopathies dans un élevage. Elle permet notamment de diminuer le nombre d'animaux atteints de myopathies sans impact négatif sur les performances de croissance de l'élevage. Ce résultat est d'autant plus surprenant que de nombreux auteurs ont décrit les conséquences négatives de la supplémentation en magnésium sur l'absorption du calcium et la résistance osseuse.

La prévention des myopathies au sens de l'invention peut correspondre à la diminution de l'incidence ou de la prévalence des myopathies observées dans l'élevage, par rapport à un élevage non supplémenté. La prévalence peut être calculée sur la base de l'ensemble des myopathies observées ou sur la base d'une partie d'entre elles seulement. Par exemple, la diminution de la prévalence des myopathies peut se traduire par l'absence de volaille souffrant d'une myopathie sévère.

Le supplément peut être utilisé dans la prévention des myopathies qui risquent d'atteindre des volailles d'élevage, notamment les poulets de chair. Autrement dit, le supplément permet de réduire le pourcentage de volailles atteintes de myopathies dans un élevage, notamment un élevage de volailles à forte croissance dans lequel la prévalence des maladies est très élevée. Par conséquent, l'invention trouve avantageusement application dans l'alimentation des volailles à forte croissance. Les volailles peuvent être des poulets lourds ou des dindes. On entend par « poulet lourd » au sens de l'invention, un poulet dont le poids d'abattage est supérieur ou égal à 2,0 kg en moyenne, voire supérieur ou égal à 2,4 kg en moyenne.

Les symptômes des myopathies peuvent être la présence de bandes blanches parallèles aux fibres musculaires dans les filets et les cuisses des volailles (White Striping) ou la dureté des filets des volailles (Wooden Breast). Dans un mode de réalisation particulier de l'invention, le supplément permet de réduire le nombre d'animaux atteints de White Striping, de réduire le nombre d'animaux atteints de Wooden Breast, ou de réduire à la fois le nombre d'animaux atteints de White Striping et le nombre d'animaux atteints de Wooden Breast.

Par exemple, l'incidence du White Striping peut être réduite d'au moins 30%, de préférence d'au moins 40%, voire même d'au moins 45%. L'incidence du Wooden Breast peut être réduite d'au moins 15%, de préférence d'au moins 20% et, dans certains modes de réalisation, peut être réduite d'au moins 45%.

Dans certains modes de réalisation, l'invention permet de supprimer totalement la survenue des myopathies de degrés supérieurs ou égaux à 3 sur une échelle à 4 points ou une échelle à 5 points. L'homme du métier saura évaluer le degré de sévérité des myopathies Wooden Breast et White Striping sur la base de ses connaissances générales, notamment en suivant l'indexation des échelles établies par Kuttappan et al., 2012 Poultry Science 91 :1240-1247, Kuttappan et al., 2016 Poultry Science 0:1-10 et Tijare et al., 2016 Poultry Science 0 :1-7. Ces échelles comportent plusieurs points, par exemple quatre points allant de 0 (absence de myopathie) à 3 (myopathie sévère).

La diminution de l'incidence des myopathies pourra être observée notamment par rapport à un groupe contrôle pour lequel la ration n'est pas supplémentée avec une source de magnésium, ledit groupe contrôle comprenant au moins 20%, au moins 30%, voire au moins 40% de volailles souffrant de myopathies à l'abattage. Le pourcentage de diminution des myopathies dépendra de l'indice de sévérité des myopathies et de la nature des myopathies.

Pourront être retenus, selon les cas, pour le calcul de la diminution des myopathies, l'ensemble des myopathies ayant un degré de sévérité allant de 1 à 4, l'ensemble des myopathies ayant un degré de sévérité allant de 2 à 4, l'ensemble des myopathies ayant un degré de sévérité de 3 ou 4, ou uniquement les myopathies d'indice 4 sur une échelle à 5 points. Pourront être retenus, selon les cas, pour le calcul de la diminution des myopathies, l'ensemble des myopathies ayant un degré de sévérité allant de 1 à 3, l'ensemble des myopathies ayant un degré de sévérité allant de 2 à 3, ou uniquement les myopathies d'indice 3 sur une échelle à 4 points. Le pourcentage de myopathies d'au moins 20% peut désigner le pourcentage de White Striping, le pourcentage de de Wooden Breast, ou la somme du pourcentage de White Striping et du pourcentage de de Wooden Breast.

Dans certains modes de réalisation, le supplément alimentaire de l'invention permet de réduire d'au moins 50% le nombre de volailles ayant des filets touchés par le Wooden Breast (tous degrés de sévérité confondus), et de réduire d'au moins 50% le nombre de volailles ayant des filets touchés par le White Striping (tous degrés de sévérité confondus), notamment dans une population comprenant 40% de poulets souffrant de myopathies.

Dans certains modes de réalisation de l'invention, le traitement permet de supprimer l'apparition de degrés sévères de Wooden Breast, par exemple les degrés supérieurs ou égaux à 3.

La diminution de l'incidence de myopathies est avantageusement obtenue sans diminution des performances de croissance des animaux, et notamment sans diminution du poids d'abattage moyen de l'élevage. L'invention peut réduire d'au moins 50% le nombre de filets déclassés en raison de myopathie, et/ou d'augmenter d'au moins 10% le nombre de filets totalement exempts de myopathies. Enfin, l'invention peut améliorer la couleur des filets et/ou la capacité de rétention en eau des filets.

La ou les sources de magnésium peuvent être choisies parmi les sels, les hydrates ou les chélates du magnésium (Mg2+). Elles peuvent être d'origine naturelle ou synthétique.

Parmi les sels de magnésium, sont recensés notamment l'oxyde de magnésium, le carbonate de magnésium, le chlorure de magnésium, l'ascorbate de magnésium, l'ascorbo-aspartate de magnésium, le L-asparatate de magnésium, le sulfate de magnésium, le stéarate de magnésium, le silicate de magnésium, le pidolate de magnésium, le phosphate de magnésium, l'acétate de magnésium, l'orotate de magnésium, le malate de magnésium, le lactate de magnésium, l'hydroxyde de magnésium, le glycérophosphate de magnésium, le gluconate de magnésium, le citrate de magnésium, le nitrate de magnésium, le nitrure de magnésium, l'oléate de magnésium, le palmitate de magnésium, le sulfonate de magnésium, le carboxylate de magnésium, le borate de magnésium, le bromure de magnésium, le chromate de magnésium, le bisulfite de magnésium, le fluorure de magnésium, l'iodate ou l'iodure de magnésium, le permanganate de magnésium, l'aluminate de magnésium, le salicylate de magnésium, le formate de magnésium, le tungstate de magnésium.

Les hydrates de magnésium au sens de l'invention désignent l'ensemble des formes hydratés de tout sel de magnésium énoncé précédemment.

Parmi les chélates de magnésium, sont présents notamment le glycinate de magnésium, bisglycinate de magnésium, l'aspartate de magnésium, l'alanate de magnésium, le glutamate de magnésium, le diglutamate de magnésium, le lysinate de magnésium, le méthionate de magnésium, le thréonate de magnésium.

Par exemple, la source de magnésium est l'oxyde de magnésium. L'oxyde de magnésium peut être obtenu par extraction minière, broyage et calcination d'une roche contenant du carbonate de magnésium. La température de calcination de la roche peut être comprise entre 550°C et 1300°C. La granulométrie de l'oxyde de magnésium obtenu peut être comprise entre 0,1 et 8 mm, et sa pureté peut aller de 70% à 99,9%.

La source de magnésium est de préférence administrée en une quantité équivalente à une dose en magnésium (Mg2+) allant 0,01% à 0,4% en masse, de préférence allant de 0,05% à 0,2% en masse par rapport à la masse de matière sèche de la ration alimentaire donnée aux volailles.

La demande décrit par ailleurs
- un supplément alimentaire comprenant au moins une source de magnésium pour l'élevage de volailles à forte croissance,
- un procédé d'élevage de volailles à forte croissance consistant à ajouter dans la ration alimentaire desdites volailles de l'élevage un additif constitué d'au moins une source de magnésium, et
- l'utilisation d'un supplément alimentaire constitué d'au moins une source de magnésium pour l'alimentation de volailles à forte croissance.

Les volailles à forte croissance sont notamment des poulets lourds tels que définis plus haut.

L'invention est illustrée plus en détail par les exemples suivants.

### Exemple 1 :

L'effet d'une supplémentation en oxyde de magnésium sur le degré d'incidence des myopathies de poulets de chair a été étudié.

### Conditions de réalisation de l'étude :

Pour cela, 270 poulets (mâles et femelles mélangés) de souche Ross 308 ont été répartis aléatoirement comme suit : un groupe contrôle et un groupe recevant 0.62% d'oxyde de magnésium. Les animaux ont reçu le même aliment sur la période de démarrage. Le traitement a été appliqué pendant les phases de croissance et finition c'est-à-dire du jour 15 au jour 42 d'élevage. L'aliment de base a été formulé pour atteindre ou dépasser les besoins recommandés par le NRC (1994). L'eau et l'aliment ont été distribués à volonté. La température a été fixée à 33 - 35°C au début de l'étude et diminuée de 3°C par semaine pour atteindre 24°C à partir du 21 ème jour de vie. 48 heures avant abattage, un stress thermique a été appliqué à l'ensemble des traitements (augmentation de la température à 30°C pendant 8h par jour avec un intervalle de 3-4 heures).

### Evaluations :

Les performances de croissance ont été enregistrées aux jours 0, 14, 28 et 42. A la fin de l'étude, 32 poulets ont été sélectionnés dans chaque groupe pour avoir des poids homogènes à l'abattage (≈ 2.4 kg ± 0.2 kg). Les filets de ces poulets ont été retirés et immédiatement transférés pour le scoring des myopathies Wooden Breast et White Striping selon une grille en 5 points. Des paramètres de qualités de la viande ont été enregistrés tels que la rétention en eau (par méthode de centrifugation selon Carvalho et al (2017)), le pH (méthode AOAC) et la couleur (réalisée avec un colorimètre MINOLTA et utilisant les indicateurs redness, lightness et yellowness).

### Résultats :

La supplémentation en magnésium n'a eu d'influence négative ni sur les performances de croissance, ni sur la santé, ni sur la mortalité. Elle a permis de diminuer l'incidence de White Striping de 43% (tous degrés de sévérité confondus) et l'incidence de Wooden Breast de 50% (tous degrés de sévérité confondus) comparé au groupe contrôle. La supplémentation en magnésium a eu un effet positif significatif sur la couleur de la viande et la capacité de rétention en eau comparé au groupe contrôle. Les filets ayant reçu la supplémentation en magnésium était plus rouges (la valeur Redness pour le groupe témoin et supplémenté était de 3.81 et 4.06 respectivement), moins pâles (la valeur Lightness pour le groupe témoin et supplémenté était de 51.76 et 53.89 respectivement) et moins jaunes (la valeur Yellowness pour le groupe témoin et supplémenté était de 2.18 et 1.34 respectivement). La capacité de rétention en eau (WHC) pour le groupe témoin et supplémenté était de 88.67 et 94.37 respectivement.

### Exemple 2:

L'effet d'une supplémentation en oxyde de magnésium sur la qualité de viande de poulets de chair a été étudié.

### Conditions de réalisation de l'étude :

Pour cela, 200 poulets (mâles uniquement) de souche Ross 308 ont été répartis aléatoirement en deux traitements expérimentaux groupe contrôle, et traitement avec 0.3% oxyde de magnésium. Les animaux ont reçu le même aliment sur la période de démarrage. Le traitement a été appliqué pendant les phases de croissance et finition c'est-à-dire du jour 15 au jour 43 d'élevage. L'aliment de base a été formulé pour atteindre ou dépasser les besoins recommandés par l'entreprise de génétique. L'eau et l'aliment ont été distribués à volonté.

### Évaluation :

Les performances de croissance ont été enregistrées aux jours 0, 12, 21, 28, 35 et 43. A la fin de l'étude, 50 poulets par groupe ont été sélectionnés pour avoir des poids homogènes à l'abattage (≈ 3.15 kg ± 0.3 kg). Les filets de ces poulets ont été retirés et immédiatement transférés pour le scoring des myopathies Wooden Breast et White Striping selon une grille en 3 points. Des paramètres de qualités de la viande ont été enregistrés tels que la capacité de rétention en eau (méthode exsudat), le pH à 24h et à 7 jours post-mortem et la couleur (chromamètre Minolta CR400 utilisant les indicateurs L*, a* et b*).

### Résultats :

La supplémentation en magnésium n'a eu d'influence négative ni sur les performances de croissance des animaux, ni sur la santé, ni sur la mortalité. Le traitement a permis de réduire l'incidence de White Striping (tous degrés de sévérité confondus) de 52%. Il a également permis de réduire l'incidence de Wooden Breast (tous degrés de sévérité confondus) de 21%. A noter que le traitement a permis de supprimer l'apparition de degrés sévères de Wooden Breast. Le traitement a notamment permis de réduire de 62% le nombre de filets déclassé à cause des myopathies et d'augmenter de 11% de nombre de filets totalement exempts de myopathies.

Le pourcentage d'exsudation pour le groupe témoin et le groupe supplémenté était de 3.7 et 3.5% respectivement. La valeur L* pour le groupe témoin et le groupe supplémenté était de 50.4 et 51.7 respectivement. La valeur b* pour le groupe témoin et le groupe supplémenté était de 0.0 et 0.8 respectivement.

### Exemple 3:

L'effet d'une supplémentation en oxyde de magnésium sur la tendreté des filets de poulets de chair a été étudié.

### Conditions de réalisation de l'étude :

Pour cela, 400 poulets (mâles uniquement) de souche Ross 308 ont été répartis aléatoirement en deux traitements expérimentaux groupe contrôle, et traitement avec 0.3% oxyde de magnésium. Les animaux ont reçu le même aliment sur la période de démarrage. Le traitement a été appliqué pendant les phases de croissance et finition c'est-à-dire du jour 15 au jour 45 d'élevage. L'aliment de base a été formulé pour atteindre ou dépasser les besoins recommandés par l'entreprise de génétique. L'eau et l'aliment ont été distribués à volonté.

### Évaluation :

Les performances de croissance ont été enregistrées aux jours 0, 12, 21, 28, 35 et 45. A la fin de l'étude, 40 poulets par groupe ont été sélectionnés pour avoir des poids homogènes à l'abattage (≈ 3.68 kg ± 0.1 kg). Les filets de ces poulets ont été retirés pour la mesure de la tendreté du filet (réalisée le jour suivant l'abattage). La mesure a été réalisée par une méthode physique utilisant une machine de compression (3 répétitions par filet) pour mesurer deux paramètres : la force de compression des filets et la dureté des filets (force de compression en fonction de la hauteur du filet).

### Résultats :

La supplémentation en magnésium n'a eu d'influence négative ni sur les performances de croissance des animaux, ni sur la santé, ni sur la mortalité. Le traitement a permis d'améliorer la tendreté des filets. La force de compression pour le groupe témoin et le groupe supplémenté était de 9.93 N et 8.13 N respectivement. La dureté des filets pour le groupe témoin et le groupe supplémenté était de 3.35 N/mm et 2.74 N/mm respectivement.

## Revendications

1. Supplément alimentaire comprenant au moins une source de magnésium choisie parmi les sels, les hydrates ou les chélates du magnésium, pour son utilisation dans la prévention des myopathies chez les volailles d'élevage.

2. Supplément alimentaire pour son utilisation selon la revendication 1, **caractérisé en ce que** les volailles sont des poulets lourds ou des dindes.

3. Supplément alimentaire pour son utilisation selon la revendication 1, **caractérisé en ce que** les symptômes des myopathies sont la présence de bandes blanches parallèles aux fibres musculaires dans les filets et les cuisses des volailles (White Striping).

4. Supplément alimentaire pour son utilisation selon la revendication 1, **caractérisé en ce que** les symptômes des myopathies sont la dureté des filets des volailles (Wooden Breast).

5. Supplément alimentaire pour son utilisation selon la revendication 1, **caractérisé en ce que** la source de magnésium est l'oxyde de magnésium.

6. Supplément alimentaire pour son utilisation selon la revendication 5, **caractérisé en ce que** l'oxyde de magnésium est obtenu par extraction minière, broyage et calcination d'une roche contenant du carbonate de magnésium.

7. Supplément alimentaire pour son utilisation selon la revendication 5 ou 6, **caractérisé en ce que** la granulométrie de l'oxyde de magnésium est comprise entre 0,1 et 8 mm, et que sa pureté va de 70 à 99,9%.

8. Supplément alimentaire pour son utilisation selon la revendication 1, **caractérisé en ce que** la source de magnésium est administrée en une quantité équivalente à une dose en magnésium allant de 0,01% à 0,4% en masse, de préférence allant de 0,05% à 0,4% en masse par rapport à la masse de matière sèche de la ration alimentaire donnée aux volailles.

## Patentansprüche

1. Futtermittelergänzung, umfassend zumindest eine Magnesiumquelle, die aus den Salzen, den Hydraten oder den Chelaten von Magnesium ausgewählt ist, zu ihrer Verwendung in der Vorbeugung von Myopathien bei Mastgeflügel.

2. Futtermittelergänzung zu ihrer Verwendung nach Anspruch 1, **dadurch gekennzeichnet, dass** das Geflügel aus schweren Hühnerrassen oder Truthähnen besteht.

3. Futtermittelergänzung zu ihrer Verwendung nach Anspruch 1, **dadurch gekennzeichnet, dass** die Symptome der Myopathien das Vorliegen von parallelen weißen Streifen in den Muskelfasern der Filets und der Schenkel des Geflügels (sog. White Striping) sind.

4. Futtermittelergänzung zu ihrer Verwendung nach Anspruch 1, **dadurch gekennzeichnet, dass** die Symptome der Myopathien die Verhärtung der Filets des Geflügels (sog. Wooden Breast) sind.

5. Futtermittelergänzung zu ihrer Verwendung nach Anspruch 1, **dadurch gekennzeichnet, dass** die Magnesiumquelle das Magnesiumoxid ist.

6. Futtermittelergänzung zu ihrer Verwendung nach Anspruch 5, **dadurch gekennzeichnet, dass** das Magnesiumoxid durch Abbau, Vermahlung und Kalzinierung eines Gesteins erhalten wird, das Magnesiumcarbonat enthält.

7. Futtermittelergänzung zu ihrer Verwendung nach Anspruch 5 oder 6, **dadurch gekennzeichnet, dass** die Korngröße des Magnesiumoxids zwischen 0,1 und 8 mm liegt, und dass seine Reinheit von 70 bis 99,9 % reicht.

8. Futtermittelergänzung zu ihrer Verwendung nach Anspruch 1, **dadurch gekennzeichnet, dass** die Magnesiumquelle in einer Menge verabreicht wird, die zu einer Magnesiumdosis von 0,01 bis 0,4 Massen-%, vorzugsweise von 0,05 bis 0,4 Massen-% bezogen auf die Trockenmasse der an das Geflügel verfütterten Futtermenge äquivalent ist.

## Claims

1. A food supplement comprising at least one source of magnesium that is chosen from magnesium salts, hydrates or chelates, for the use thereof in the prevention of myopathies in farmed poultry.

2. The food supplement for the use as claimed in claim 1, **characterized in that** the poultry are heavy chickens or turkeys.

3. The food supplement for the use as claimed in claim 1, **characterized in that** the symptoms of the myopathies are the presence of white bands parallel to the muscle fibers in the fillets and the thighs of the poultry (white striping).

4. The food supplement for the use as claimed in claim 1, **characterized in that** the symptoms of the myopathies are hardness of the fillets of the poultry (wooden breast).

5. The food supplement for the use as claimed in claim 1, **characterized in that** the source of magnesium is magnesium oxide.

6. The food supplement for the use as claimed in claim 5, **characterized in that** the magnesium oxide is obtained by mining, grinding and calcining a rock containing magnesium carbonate.

7. The food supplement for the use as claimed in claim 5 or 6, **characterized in that** the particle size of the magnesium oxide is between 0.1 and 8 mm, and **in that** its purity ranges from 70% to 99.9%.

8. The food supplement for the use as claimed in claim 1, **characterized in that** the source of magnesium is administered in an amount equivalent to a magnesium dose ranging from 0.01% to 0.4% by mass, preferably ranging from 0.05% to 0.4% by mass, relative to the mass of solids in the feed ration given to the poultry.
